# EUROPÄISCHE PATENTANMELDUNG

(11) **EP 1 279 659 A2**
(43) Veröffentlichungstag der Anmeldung: **29.01.2003**
(21) Anmeldenummer: 02015584.2
(22) Anmeldetag: 15.07.2002
(51) Int. Cl.: C07C 68/00, C07C 69/96

(54) **Verfahren zur Herstellung von Diarylcarbonaten**

(30) Priorität: 27.07.2001 DE 10136856
(71) Anmelder: BAYER AG, 51368 Leverkusen (DE)
(72) Erfinder: Reisinger, Claus-Peter, Dr., Wixom, MI 48393 (US); Hansen, Sven Michael, Dr., 47829 Krefeld (DE); Fischer, Peter, Dr., 50676 K-ln (DE)

(57) **Zusammenfassung**

Die Erfindung betrifft ein Verfahren zur Herstellung von Diarylcarbonaten durch Direktcarbonylierung von Hydroxyaromaten, das dadurch gekennzeichnet ist, dass bestimmte Komponenten während der Reaktion nachdosiert werden.

## Beschreibung

Die vorliegende Anmeldung betrifft ein Verfahren zur Herstellung von Diarylcarbonaten (DAC) dadurch gekennzeichnet, dass bestimmte Komponenten zu bestimmten Zeitpunkten, abhängig von der Reaktionsführung, nachdosiert werden.

Die Herstellung von DAC durch oxidative Direktcarbonylierung von aromatischen Hydroxyverbindungen in Gegenwart von CO, O₂ und eines Edelmetall-Katalysators ist bekannt (siehe z.B. DE-OS 27 38 437, US-A 4,349,485, US-A 5,231,210, EP-A 667 336, EP-A 858 991, US-A 5,760,272). Als Edelmetall wird bevorzugt Palladium eingesetzt. Zusätzlich können ein Cokatalysator (z. B. Mangan- oder Kobaltsalze), eine Base, Bromidquellen, quaternäre Salze, verschiedene Chinone bzw. Hydrochinone und Trockenmittel eingesetzt werden. Dabei kann in einem Lösungsmittel gearbeitet werden.

Die bekannten Verfahren liefern jedoch keine für eine technische Umsetzung zufriedenstellenden Ausbeuten und erzeugen größere Mengen Nebenprodukte. Es ist daher wünschenswert ein Verfahren bereitzustellen, welches in bezug auf Ausbeute und Produktqualität optimiert ist.

Es wurde nun ein Verfahren gefunden, welches eine überraschende Steigerung der Selektivität der Reaktion, d.h. die Abnahme von Nebenprodukten bei gleichbleibender Produktausbeute ermöglicht, dadurch gekennzeichnet, daß bestimmte Komponenten nachdosiert werden.

Gegenstand der Erfindung ist somit ein Verfahren zur Herstellung eines aromatischen Carbonats der Formel

R-O-CO-O-R (I),

worin
- R: für gegebenenfalls substituierte, aromatische Kohlenwasserstoffreste, bevorzugt mit 6 bis 22 Kohlenstoffatomen steht,
bei dem eine aromatischen Hydroxyverbindung der Formel

R-O-H (II),

worin
- R: die gleich Bedeutung wie oben hat,

CO und O2, gegebenenfalls in einem Lösungsmittel in Anwesenheit der Komponenten
a) ein Metallsalz der Gruppe VIIIB,
b) mindestens ein zweites Metallsalz,
c) ein Bromidsalz
d) sowie eine Base
zur Reaktion gebracht werden, dadurch gekennzeichnet, dass eine oder mehrere der Komponenten a-d) mindestens einmal während der Reaktionszeit in die Reaktionsmischung nachdosiert werden.

Bei den erfindungsgemäß umsetzbaren aromatischen Hydroxyverbindungen R-O-H handelt es sich beispielsweise um Monohydroxyverbindungen wie Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol oder Di- bzw. Polyhydroxyverbindungen wie Resorcin und Hydrochinon, sowie Tris- und Bisphenole wie 2,2-Bis-(4-hydroxyphenyl)-propan (Bisphenol A), 2,2- Bis-(3,5-dimethyl-4-hydroxyphenyl)-propan, 1,1-Bis-(4-hydroxyphenyl)-cyclohexan, 1,1-Bis-(4-hydroxyphenyl)-3,3,5-trimethylcyclohexan oder 6,6'-Dihydroxy-3,3,3',3'-tetramethyl-1,1'-spiro(bis)-indan, 2,4'-Hydroxybiphenyl oder 4,4'-Hydroxybiphenyl. Allgemein handelt es sich im Falle einer Substitution der aromatischen Hydroxyverbindung um 1 bis 3 Substituenten in der Bedeutung von C₁-C₁₈-Alkyl, C₆-C₁₈-Aryl, C₇-C₁₈-Aralkyl, C₁-C₁₈-Alkoxy, Fluor, Chlor oder Brom. Bevorzugt werden Monohydroxyverbindungen eingesetzt, besonders bevorzugt Phenol.

Die für das erfindungsgemäße Verfahren geeigneten Platinmetall-Katalysatoren a) bestehen aus mindestens einem Edelmetall der Gruppe VIIIB, vorzugsweise Palladium. Es kann bei dem erfindungsgemäßen Verfahren in verschiedener Form zugegeben werden. Palladium kann in metallischer Form z.B. als Palladiumschwarz oder auf einem Träger wie Pd/C, Pd/Al₂O₃, Pd/SiO₂ oder bevorzugt in Form von Palladium-Verbindungen der Oxidationsstufen 0 und +2, wie beispielsweise Palladium(II)-acetylacetonat, -halogenide, -carboxylate von C₂-C₁₈-Carbonsäuren, -dicarboxylate wie Oxalat, -nitrat, -sulfat, -oxide oder Palladiumkomplexe, die beispielsweise Kohlenmonoxid, Olefine, Amine, Nitrile, Phosphorverbindungen und Halogenide enthalten können, eingesetzt werden. Besonders bevorzugt sind Palladiumbromid und Palladiumacetylacetonat.

Die Menge an Platinmetall-Katalysator ist im erfindungsgemäßen Verfahren nicht beschränkt. Bevorzugt wird so viel Katalysator zugesetzt, dass die Konzentration des Metalls im Reaktionsansatz 1 bis 3000 ppm beträgt, besonders bevorzugt sind Konzentrationen von 5 bis 500 ppm.

Als zweites, als Cokatalysator wirkendes, Metallsalz b) für das erfindungsgemäße Verfahren wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew), ggf. auch Mischungen davon, verwendet, wobei das Metall in verschiedenen Oxidationsstufen eingesetzt werden kann. (s. z. B. US-A 5,142,086, US-A 5,231,210, US-A 5,284,964, EP-A 350 697, EP-A 350 700, US-A 5,336,803) Bevorzugt werden Pb, Ti, Mn, Cu, Co, V, Zn, Ce und Mo eingesetzt. Ohne das erfindungsgemäße Verfahren einzuschränken, seien Blei (II), Mangan(II), Mangan(III), Kupfer(I), Kupfer(II), Kobalt(II), Kobalt(III), Vanadium(III) und Vanadium(IV), insbesondere Mangan(II), Mangan(III), Kobalt(II), Kobalt(III), genannt. Die Metalle können beispielsweise als Halogenide, Oxide, Carboxylate von C₂-C₁₈-Carbonsäuren, Diketonate oder Nitrate sowie als Komplexverbindungen eingesetzt werden, die beispielsweise Kohlenmonoxid, Olefine, aromatische und aliphatische Mono-oder Polyamine, Phosphorverbindungen, Pyridine, Bipyridine, Terpyridine, Chinoline, Isochinoline, Kryptanden, Schiffbasen und Halogenide enthalten können. Besonders bevorzugt werden Mn, Cu, Mo, Pb und Ce eingesetzt. Ganz besonders bevorzugt werden Manganverbindungen im erfindungsgemäßen Verfahren verwendet, besonders bevorzugt Mangan(II)- und Mangan(III)komplexe, ganz besonders bevorzugt Mangan(II)acetylacetonat bzw. Mangan(III)-acetylacetonat, sowie Mangan(II)bromid.

Der Cokatalysator, der auch in-situ gebildet werden kann, wird in einer solchen Menge zugesetzt, dass seine Konzentration im Bereich von 0,0001 bis 20 Gew.% des Reaktionsgemisches liegt, bevorzugt ist der Konzentrationsbereich von 0,001 bis 5 Gew.%, besonders bevorzugt 0,005 bis 2 Gew.%.

Bei den im Rahmen der vorliegenden Erfindung eingesetzten Bromidverbindungen c) handelt es sich beispielsweise um die Alkalibromide oder Erdalkalibromide, bevorzugt aber um die Bromidsalze von organischen Kationen. Bei den organischen Kationen kann es sich beispielsweise um mit organischen Resten substituierte Ammonium-, Guanidinium-, Phosphonium- oder Sulfoniumsalze, ggf. auch Mischungen davon, handeln. Geeignet für den Einsatz im erfindungsgemäßen Verfahren sind Ammonium-, Guanidinium-, Phosphonium- und Sulfoniumsalze, die als organische Reste C₆- bis C₁₀-Aryl-, C₇- bis C₁₂-Aralkyl- und/oder C₁- bis C₂₀-Alkyl-Reste enthalten. Bevorzugt werden in das erfindungsgemäße Verfahren Ammoniumsalze eingesetzt, die als organische Reste C₆- bis C₁₀-Aryl-, C₇- bis C₁₂-Aralkyl- und/oder C₁- bis C₂₀-Alkyl-Reste tragen, besonders bevorzugt sind Tetrabutylammoniumbromid und Tetrabutylphosphoniumbromid. Die Menge eines solchen quaternären Salzes kann beispielsweise 0,1 - 20 Gew.%, bezogen auf das Gewicht des Reaktionsgemisches, betragen. Vorzugsweise beträgt diese Menge 0,5 bis 15 Gew.%, besonders bevorzugt 1 bis 5 Gew.%.

Für das erfindungsgemäße Verfahren einsetzbare Basen sind Alkalihydroxide, Alkalisalze bzw. quaternäre Salze von schwachen Säuren wie Alkali-tert.-butylate oder Alkalisalze bzw. quaternäre Salze von aromatischen Hydroxyverbindungen der Formel (II), in der R die oben angegebene Bedeutung hat. Ganz besonders bevorzugt wird ein Alkalisalz bzw. quaternäres Salz der aromatischen Hydroxyverbindung der Formel (II) verwendet, die auch zum organischen Carbonat umgesetzt werden soll, beispielsweise Tetrabutylammonium- oder Kaliumphenolat.

Die Alkalisalze können Lithium-, Natrium-, Kalium-, Rubidium- oder Cäsiumsalze sein. Bevorzugt werden Lithium-, Natrium-, und Kaliumphenolate, besonders bevorzugt Kaliumphenolat eingesetzt.

Die quaternären Salze können Ammonium-, Phosphonium-, Pyridinium-, Sulfonium oder Guanidiniumsalze sein, die als organische Reste C₆- bis C₁₈-Aryl-, C₇- bis C₁₈-Aralkyl- und/oder C₁- bis C₂₀-Alkyl-Reste besitzen. Die Reste können alle gleich oder verschieden sein, ggf. können auch Mischungen mehrerer Quartärsalze eingesetzt werden. Bevorzugt wird dabei ggf. dasselbe Kation eingesetzt, das auch als Bromid für Komponente c) verwendet wird. Weiterhin bevorzugt werden Tetraphenylphosphonium, Tetrabutylammonium-, Tetrabutylphosphonium, besonders bevorzugt wird Tetrabutylammonium.

Alternativ können auch Trialkylaminbasen wie Tributylamin, Diisopropylethylamin, DBU, DBN verwendet werden.

Die Base d) wird in einer von der Stöchiometrie unabhängigen Menge zugesetzt. Das Verhältnis von Platinmetall, z.B. Palladium, zu Base wird vorzugsweise so gewählt, dass pro mol Platinmetall, z.B. Palladium, 0,1 bis 5000, bevorzugt 1 bis 1000, besonders bevorzugt 10 bis 300 Äquivalente Base eingesetzt werden.

Die erfindungsgemäße Nachdosierung kann für eine oder mehrere der Katalysatorkomponenten a) bis d) erfolgen, bevorzugt wird jedoch die Nachdosierung der Katalysatorkomponenten a) und/oder b).
Besonders bevorzugt werden dabei Pd-Verbindungen und/oder Mangansalze b) nachdosiert.

Die Katalysatorkomponenten können portionsweise ein- oder mehrmals nachdosiert werden. Bevorzugt wird dabei mit einer innerhalb der ersten Stunde Reaktionszeit eingestellten Startkonzentration von etwa 10 bis 80 Gew.% der Gesamtmenge begonnen und die Restmenge in einem oder mehreren Intervallen zugegeben. Besonders bevorzugt beträgt die Startkonzentration etwa 30 bis 70 Gew.%. Bevorzugt wird die Zugabe in einer bis etwa zehn Portionen. Die Zeitintervalle werden bevorzugt etwa äquidistant gewählt. Bevorzugt wird eine Zugabe der letzten Portion, zu einem Zeitpunkt, der vor etwa 90 %, besonders bevorzugt vor etwa 80 % der gesamten Reaktionszeit, bzw. mittleren Verweilzeit liegt.

Die nachdosierte Menge der Katalysatorkomponente kann auch kontinuierlich eingeleitet werden, beispielsweise durch langsames Zupumpen einer Lösung. Bevorzugt wird dabei mit einer innerhalb der ersten Stunde Reaktionszeit eingestellten Startkonzentration von etwa 10 bis 80 Gew.% der Gesamtmenge begonnen und die Restmenge anschließend kontinuierlich zudosiert. Besonders bevorzugt beträgt die Startkonzentration etwa 30 bis 70 Gew.%. Dabei können verschiedene Zeit-Konzentrationsdosierprofile eingestellt werden, z.B. kann am Anfang eine größere Menge pro Zeiteinheit zudosiert werden als zum Ende der Reaktionszeit hin oder umgekehrt. Bevorzugt wird eine zeitlineare Zugabe der Restkatalysatormenge, bei der die zugegebene Menge pro Zeitintervall in etwa konstant bleibt. Die Zugabe kann zu beliebigen Zeitpunkten während der Reaktionsdauer, bzw. der mittleren Verweilzeit begonnen und abgebrochen werden.

Bevorzugt wird eine Beendigung der Dosierung zu einem Zeitpunkt, der vor etwa 90 %, besonders bevorzugt vor etwa 80 % der gesamten Reaktionszeit, bzw. mittleren Verweilzeit liegt.

Das erfindungsgemäße Verfahren kann dabei sowohl in kontinuierlicher wie auch diskontinuierlicher Weise geführt werden.

Bei einer diskontinuierlichen Durchführung wird die Katalysatorkomponente zu einem Zeitpunkt nach Beginn der Reaktion im Reaktor zudosiert.

Bei einer kontinuierlichen Durchführung können beispielsweise in einer Kaskade von n Reaktionsapparaten eine oder mehrere der Komponenten a-d) während der Reaktion in mindestens einen der 2. bis n.ten Reaktoren zudosiert werden.

Das erfindungsgemäße Verfahren zur Carbonatbildung wird bei einer Reaktionstemperatur von 30 bis 200°C, bevorzugt 50 bis 150°C, besonders bevorzugt 60 bis 130°C, und bei einem Reaktionsdruck von 1 bis 200 bar, bevorzugt 1 bis 50 bar, besonders bevorzugt 1 bis 10 bar durchgeführt.

Als inertes organisches Lösungsmittel können Kohlenwasserstoffe, halogenierte Kohlenwasserstoffe und aromatische Lösemittel wie Chlorbenzol, Dichlorbenzol, Fluorbenzol, Benzol, Toluol, Anisol, Cyclohexan, Petrolether, Methylenchlorid oder 1,2-Dichlorethan, dipolar aprotische Lösungsmittel wie Dimethylacetamid, Acetonitril, N-Methylpyrrolidinon, Ether wie Dioxan, Tetrahydrofuran, t-Butylmethylether und veretherte Glykole, ggf. auch Mischungen verschiedener Lösungsmittel verwendet werden. Besonders bevorzugt wird Chlorbenzol eingesetzt. Das inerte Lösemittel kann mit einem Anteil von 1 bis 99 %, bevorzugt 20 bis 98 %, besonders bevorzugt 40 bis 98 %, in der Reaktionsmischung enthalten sein.

Die Zusammensetzung der Reaktionsgase Kohlenmonoxid und Sauerstoff kann in weiten Konzentrationsgrenzen variiert werden, es wird jedoch zweckmäßigerweise ein CO:O₂-Molverhältnis (normiert auf CO) von 1:0,001 bis 1:1, bevorzugt 1:0,01 bis 1:0,5 und besonders bevorzugt von 1:0,02 bis 1:0,3 eingestellt. Der Sauerstoffpartialdruck ist bei diesen Molverhältnissen groß genug, um hohe Raum-Zeit-Ausbeuten erreichen zu können.

Alle Ausgangsverbindungen können mit Verunreinigungen aus ihrer Herstellung und Lagerung kontaminiert sein, jedoch ist es im Sinne der Reinheit des Endproduktes wünschenswert, mit möglichst sauberen Chemikalien zu arbeiten. Auch die Reaktionsgase unterliegen keinen besonderen Reinheitsanforderungen. So kann Synthesegas als CO-Quelle und Luft als O₂-Träger dienen, es ist jedoch darauf zu achten, dass keine Katalysatorgifte wie z.B. Schwefel oder dessen Verbindungen eingetragen werden. Die Gase können mit einem oder mehreren anderen Gasen wie Stickstoff, Argon, Kohlendioxid oder Wasserstoff verdünnt sein. In der bevorzugten Ausführungsform des erfindungsgemäßen Verfahrens werden reines CO und reiner Sauerstoff verwendet.

In einer weiteren Ausführungsform werden statt des homogenen Katalysatorsystems heterogene Katalysatoren, bei denen das Platinmetall oder das Platinmetall und/oder der Cokatalysator auf einem heterogenen Träger aufgebracht sind, als Pulver oder Formkörper eingesetzt. Die übrigen Komponenten des Katalysatorsystems, wie die Base, die quaternäre Verbindung und gegebenenfalls der Cokatalysator, sind weiterhin in der Reaktionslösung homogen gelöst. Die Menge des Platinmetalls am Gesamtgewicht des heterogenen Katalysators beträgt 0,01 bis 15 Gew.%, bevorzugt 0,05 bis 10 Gew.%, gerechnet als Platinmetall.

Als Cokatalysatoren auf dem Katalysatorträger wird mindestens eine Metallverbindung der oben genannten Art eingesetzt.

Der Anteil des Cokatalysators am Gesamtgewicht des heterogenen Katalysators beträgt 0,01 bis 15 Gew.%, bevorzugt 0,05 bis 10 Gew.% gerechnet als Metall.

Als Katalysatorträger eignen sich ein oder mehrere Metalloxide aus der Gruppe von V, Mn, Ti, Cu, Zr, La, der Seltenerdmetalle (Atomnummern 58-71), sowohl im Sinne chemisch einheitlicher Reinsubstanzen als auch im Gemisch sowie Eisen- und Kobaltoxide, Nickel-, Aluminium-, Silizium- und Magnesiumoxid, Zeolithe und Aktivkohlen. Wird der Trägerkatalysator als Pulver eingesetzt, sind zur Vermischung der Reaktionskomponenten die zu verwendenden Rührbehälter mit dafür brauchbaren Rührern ausgestattet, bzw. als Blasensäulenreaktor gestaltet.

Beim Arbeiten mit Trägerkatalysator-Pulvern als Suspension in Rührgefäßen oder Blasensäulen werden Mengen von 0,001 bis 50 Gew.%, bevorzugt von 0,01 bis 20 Gew.%, besonders bevorzugt von 0,1 bis 10 Gew.% Trägerkatalysator-Pulver auf die eingesetzte Menge an aromatischer Hydroxyverbindung, verwendet.

In bevorzugten Ausführungsformen wird der heterogene Trägerkatalysator ortsfest in Rührbehältern, einer Blasensäule, einem Rieselphasenreaktor oder Kaskaden dieser Reaktoren eingesetzt. Eine Abtrennung des Trägerkatalysators entfällt dann völlig.

Als Reaktoren für das erfindungsgemäße Verfahren mit homogenem oder heterogenem Katalysator sind Rührkessel, Autoklaven und Blasensäulen geeignet, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinandergeschaltet sein.

Zur Vermischung der Reaktionskomponenten sind die erfindungsgemäß zu verwendenden Rührbehälter mit dafür geeigneten Rührer ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohlrührertypen. Bevorzugte Rührer sind solche, die eine effektive Vermischung von Gasen und Flüssigkeiten erlauben, beispielsweise Hohlrohrbegasungsrührer, Propellerrührer etc.

Als Blasensäulen können in dem erfindungsgemäßen Verfahren folgende Typen, eingesetzt werden: einfache Blasensäulen, Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie z.B.: Mammutschlaufenreaktoren, Abstromschlaufenreaktoren, Strahlschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren (Chem. Ing. Tech. 51 (1979) Nr. 3, S. 208-216; W.-D. Deckwer, Reaktionstechnik in Blasensäulen, Otto Salle Verlag 1985).

In einer bevorzugten Ausführungsform kommen Blasensäulenreaktoren und Blasensäulen-Kaskaden zum Einsatz, die eine effektive Vermischung von Gas und Flüssigkeiten erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden, sowie weitere dem Fachmann bekannte Einbauten zum Einsatz. Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer wie im Falle eines Rührkessels oder durch andere bekannte Gasverteilungsorgane in die Reaktionsmischung geleitet. Nach Erreichen des optimalen Umsatzes wird das Reaktionsgemisch aus dem Reaktor entfernt oder gegebenenfalls im Reaktor aufgearbeitet. Im Falle der Verwendung pulverförmiger Trägerkatalysatoren können diese aus der Reaktionsmischung z.B. durch Filtration, Sedimentation oder Zentrifugieren abgetrennt werden.

In diskontinuierlichen Versuchen verwendete Trägerkatalysatoren können bei gleichen Einsatzstoffen ggf. ohne Reinigung wiederholt eingesetzt werden. Bei kontinuierlicher Arbeitsweise können die eingesetzten Trägerkatalysatoren über lange Zeit im Reaktor verbleiben und gegebenenfalls regeneriert werden.

In bevorzugter Weise kommt eine kontinuierliche Arbeitsweise im Einzelreaktor oder in einer Kaskade mehrerer Reaktoren zum Einsatz. Bei Verwendung ortsfester heterogener Katalysatoren können diese über lange Zeit im Reaktor verbleiben und dort auch ggf. regeneriert werden.

### Beispiele

Die Reaktionskomponenten werden durch Gaschromatographie untersucht, wobei die Massen der Komponenten mittels eines internen Standards bestimmt werden können. Die Selektivität wird berechnet, indem die Menge des in der Reaktionsmischung enthaltenen Restphenols und des zu DPC umgesetzten Phenols zusammenaddiert und durch die eingesetzte Gesamtphenolmenge dividiert werden. Das dabei nicht erfasste Phenol wurde zu Nebenprodukten umgesetzt.

Die integrale Menge der Katalysatorkomponenten ist definiert als Integral über die Konzentrations-Zeitkurve, die integrale Turn Over Frequency (TOF) ist die in 3 h insgesamt erzeugte DPC-Menge in mol dividiert durch die integrale Menge an Katalysatorkomponente.

### Beispiel 1

In einen Autoklaven werden 0,12 mmol Palladium(II)bromid, 40 mmol Tetrabutylammoniumbromid, 2,8 mmol Mangantrisacetylacetonat und 64 g Phenol in 300 g Chlorbenzol vorgelegt. Anschließend wird bei 80°C 15 min Kohlenmonoxid (<180 Nl/h) durchgeleitet, worauf 40 mmol Tetrabutylammoniumphenolat in 50 ml Chlorbenzol zugegeben werden. Bei 90°C/3 bar werden 320 Nl/h einer Gasmischung aus Kohlenmonoxid und Sauerstoff (97,5:2,5 Vol.%), durchgeleitet. Innerhalb von 120 Minuten werden zusätzlich 1,25 mmol Mangantrisacetylacetonat in 60 ml Chlorbenzol kontinuierlich und gleichmäßig zugepumpt.

Nach 1, 2 und 3 Stunden wird eine Probe genommen und mittels GC untersucht.

### Vergleichsbeispiel 1

In einen Autoklaven werden bei 80°C 0,12 mmol Palladium(II)bromid, 40 mmol Tetrabutylammoniumbromid, 4,2 mmol Mangantrisacetylacetonat und 64 g Phenol in 300 g Chlorbenzol vorgelegt. Anschließend wird bei 80°C 15 min Kohlenmonoxid (<180 Nl/h) durchgeleitet, worauf 40 mmol Tetrabutylammoniumphenolat in 50 ml Chlorbenzol zugegeben werden. Bei 90°C/3 bar werden 320 Nl/h einer Gasmischung aus Kohlenmonoxid und Sauerstoff (97,5:2,5 Vol.%), durchgeleitet. Nach 1, 2 und 3 Stunden wird eine Probe genommen und mittels GC untersucht.

**Tabelle 1:**

| Nachdosierung von Mangantrisacetylacetonat (Komponente b)) | | | | | | |
|---|---|---|---|---|---|---|
| | Zeit [h] | Mn-Menge [mmol] | Phenolselektivität [%] | DPC [g] | integrale Mn-Menge [mmol·h] | integrale TOF [mmol(DPC)/(mmol(Mn)·h)/h] |
| Vgl. 1 | 0 | 4,20 | 100,0 | 0,0 | 12,6 | 21,0 |
| | 1 | 4,20 | 94,9 | 28,0 | | |
| | 2 | 4,20 | 92,2 | 46,4 | | |
| | 3 | 4,20 | 91,6 | 56,7 | | |
| Bsp. 1 | 0 | 2,8 | 100,0 | 0,0 | 10,9 | 23,8 |
| | 1 | 3,21 | 95,8 | 22,3 | | |
| | 2 | 4,05 | 94,5 | 43,8 | | |
| | 3 | 4,05 | 93,8 | 55,6 | | |

### Beispiel 2

In einen Autoklaven werden bei 80°C 0,18 mmol Palladium(II)bromid, 40 mmol Tetrabutylammoniumbromid, 2,8 mmol Mangantrisacetylacetonat und 64 g Phenol in 300 g Chlorbenzol vorgelegt. Anschließend wird 15 min Kohlenmonoxid (<180 Nl/h) durchgeleitet, worauf 40 mmol Tetrabutylammoniumphenolat in 50 ml Chlorbenzol zugegeben werden. Bei 90°C/3 bar werden 320 Nl/h einer Gasmischung aus Kohlenmonoxid und Sauerstoff (97,5:2,5 Vol.%), durchgeleitet. Innerhalb von 120 Minuten werden zusätzlich 0,070 mmol Palladiumbromid und 0,25 mmol Tetrabutylammoniumbromid in 108 ml Chlorbenzol kontinuierlich und gleichmäßig zugepumpt. Nach 1, 2 und 3 Stunden wird eine Probe genommen und mittels GC untersucht.

### Vergleichsbeispiel 2

In einen Autoklaven werden bei 80°C 0,18 mmol Palladium(II)bromid, 40 mmol Tetrabutylammoniumbromid, 2,8 mmol Mangantrisacetylacetonat und 64 g Phenol in 300 g Chlorbenzol vorgelegt. Anschließend wird 15 min Kohlenmonoxid (<180 Nl/h) durchgeleitet, worauf 40 mmol Tetrabutylammoniumphenolat in 50 ml Chlorbenzol zugegeben werden. Bei 90°C/3 bar werden 320 Nl/h einer Gasmischung aus Kohlenmonoxid und Sauerstoff (97,5:2,5 Vol.%), durchgeleitet. Nach 1, 2 und 3 Stunden wird eine Probe genommen und mittels GC untersucht.

**Tabelle 2:**

| Nachdosierung von Palladium (Komponente a)) | | | | | | |
|---|---|---|---|---|---|---|
| | Zeit [h] | Pd-Menge [mmol] | Phenolselektivität [%] | DPC [g] | integrale Pd-Menge [mmol·h] | integrale TOF [mmol(DPC)/(mmol(Pd)·h)/h] |
| Vgl. 2 | 0 | 0,180 | 100,0 | 0,0 | 0,54 | 458,2 |
| | 1 | 0,180 | 93,9 | 24,3 | | |
| | 2 | 0,180 | 92,6 | 41,9 | | |
| | 3 | 0,180 | 91,8 | 53,0 | | |
| Bsp. 2 | 0 | 0,120 | 100,0 | 0,0 | 0,50 | 506,0 |
| | 1 | 0,161 | 93,9 | 22,6 | | |
| | 2 | 0,191 | 93,7 | 44,7 | | |
| | 3 | 0,190 | 93,3 | 54,2 | | |

### Beispiel 3

In einer kontinuierlich arbeitenden Apparatur mit 2 kaskadierten Autoklaven werden bei 96°C/3bar (Kohlenmonoxid/Sauerstoff= 97,5/2,5 Vol.%), in den ersten Autoklaven 30 mg/kg Palladium (als Palladium(II)-bromid), 250 mg/kg Mangan (als Mangan(III)trisacetylacetonat), 2 Gew.% Tetrabutylammoniumbromid, 1,8 Gew.% Tetrabutylammoniumphenolat, 16 Gew.% Phenol, sowie 74 Gew.% Chlorbenzol kontinuierlich als Feed zudosiert. Im zweiten Autoklaven wird bei 110°C zusätzlich ein Feed von 10 mg/kg Palladium in 0,18 Gew.% Tetrabutylammoniumbromid und 6 Gew.% Chlorbenzol zudosiert. Bei Verweilzeiten von je ca. 2 h pro Reaktor werden am Ausgang des zweiten Reaktors Proben entnommen und mittels GC untersucht. Die Ergebnisse zeigt Tab. 3.

### Vergleichsbeispiel 3

In einer kontinuierlich arbeitenden Apparatur mit 2 kaskadierten Autoklaven werden analog zu Beispiel 3 bei 96°C/3bar (Kohlenmonoxid/Sauerstoff= 97,5/2,5 Vol.%), in den ersten Autoklaven 40 mg/kg Palladium (als Palladium(II)-bromid), 250 mg/kg Mangan (als Mangan(III)trisacetylacetonat), 2 Gew.% Tetrabutylammoniumbromid, 1,8 Gew.% Tetrabutylammoniumphenolat und 16 Gew.% Phenol, sowie 80,2 Gew.% Chlorbenzol kontinuierlich zudosiert. Bei Verweilzeiten von je ca. 2 h pro Reaktor werden am Ausgang des zweiten Reaktors Proben entnommen und mittels GC untersucht. Die Ergebnisse zeigt Tab. 3

**Tabelle 3:**

| Nachdosierung von Palladium (Komponente a)) bei kontinuierlicher Fahrweise | | | | |
|---|---|---|---|---|
| | Laufzeit [h] | Pd-Menge [mg/kgl] | Phenolselektivität [%] | TON Pd [mmol(DPC)/(mmol(Pd)-h)/h] |
| Vgl. 3 | 21 | 40 | 84,0 | 1171 |
| | 32 | 40 | 85,0 | 1153 |
| Bsp. 2 | 22 | 30+10 | 87,3 | 1278 |
| | 30 | 30+10 | 86,8 | 1217 |

Die Beispiele zeigen, dass sich die Aktivität und Selektivität der Katalysatorkomponenten durch das erfindungsgemäße Nachdosieren überraschenderweise erhöht.

## Patentansprüche

1. Ein Verfahren zur Herstellung von Diarylcarbonaten der Formel
R-O-CO-O-R (I),
worin
R für gegebenenfalls substituierte, aromatische Kohlenwasserstoffreste, bevorzugt mit 6 bis 22 Kohlenstoffatomen steht,
bei dem eine aromatischen Hydroxyverbindung der Formel
R-O-H (II),
worin
R die gleich Bedeutung wie oben hat,
CO und O2, gegebenenfalls in einem Lösungsmittel in Anwesenheit der Komponenten
a) ein Metallsalz der Gruppe VIIIB,
b) mindestens ein zweites Metallsalz,
c) eine Bromidquelle
d) sowie eine Base
zur Reaktion gebracht werden, **dadurch gekennzeichnet, dass** eine oder mehrere der Komponenten a-d) mindestens einmal während der Reaktionszeit in die Reaktionsmischung nachdosiert werden.

2. Verfahren nach Anspruch 1, bei dem mindestens ein Metallsalz der Gruppe VIIIB nachdosiert wird.

3. Verfahren nach Anspruch 1, bei dem mindestens ein Element der Gruppe der Verbindungen von Blei, Cer, Cobalt, Kupfer, Titan, Zink, Molybdän, Mangan und Vanadium nachdosiert wird.

4. Verfahren, nach Anspruch 1, bei dem die in der ersten halben Stunde der Reaktionszeit dosierte Menge mindestens einer der Komponenten a) bis d) etwa 10 bis 80 % der zudosierten Gesamtmenge beträgt.

5. Verfahren nach Anspruch 1, bei dem es sich um die Herstellung von Diphenylcarbonat handelt.

6. Verfahren nach Anspruch 1, bei dem die Nachdosierung der Komponenten a bis d) portionsweise in ein bis zehn Portionen erfolgt.

7. Verfahren nach Anspruch 1, bei dem die Nachdosierung der Komponenten a bis d) kontinuierlich erfolgt.

8. Ein Verfahren zur kontinuierlichen Herstellung von Diarylcarbonaten der Formel
R-O-CO-O-R (I),
worin
R für gegebenenfalls substituierte, aromatische Kohlenwasserstoffreste, bevorzugt mit 6 bis 22 Kohlenstoffatomen steht,
bei dem eine aromatischen Hydroxyverbindung der Formel
R-O-H (II),
worin
R die gleich Bedeutung wie oben hat,
CO und O2, gegebenenfalls in einem Lösungsmittel in Anwesenheit der Komponenten
a) ein Metallsalz der Gruppe VIIIB,
b) mindestens ein zweites Metallsalz,
c) ein Bromidsalz
d) sowie eine Base
zur Reaktion gebracht werden, **dadurch gekennzeichnet, dass** in einer Kaskade von n Reaktionsapparaten eine oder mehrere der Komponenten a-d) während der Reaktion in mindestens einen der 2. bis n.ten Reaktoren zudosiert werden.
